# EUROPEAN PATENT SPECIFICATION

(11) **EP 3 052 108 B1**
(45) Date of publication and mention of the grant of the patent: **25.09.2024**
(21) Application number: 14793026.7
(22) Date of filing: 02.10.2014
(51) Int. Cl.: A61K 33/00, A61P 17/02

(54) **WOUND TREATMENT COMPOSITION**
WUNDBEHANDLUNGSZUSAMMENSETZUNG
COMPOSITION DE TRAITEMENT DES PLAIES

(30) Priority: 02.10.2013 EP 13004755
(43) Date of publication of application: 10.08.2016
(73) Proprietor: Special Water Patents B.V., 3903 LW Veenendaal (NL)
(72) Inventor: DE RIJK, Jan, NL-3903 LW Veenendaal (NL)
(74) Representative: Huygens, Arthur Victor
(86) International application number: PCT/EP2014/071217
(87) International publication number: WO 2015/049364

(56) References cited:
- WO-A2-2006/035320
- WO-A2-2007/099398

## Description

The present invention is defined in the appended claims. It relates to a novel wound treatment composition which is especially suitable as an rinsing agent for cleaning surfaces of the body, dissolving incrustations or scabs from body surfaces and as a dissolving agent for dressings.

It is well known in the art of dermatology and wound treatment that, especially in the case of chronic wounds, healing of wounds is strongly delayed by coatings on the wound. These coatings usually consist of wound exudate residues, of shed or enlarged fibrin coatings, necrotic tissue and cell debris. They offer favourable growth conditions for germs which, when pathogenic, may result in infection of the wound.

The biofilm mode of growth of the infecting organisms is one of the obstacles to the healing of chronic wounds (Wolcott and Rhoads, 2008). By definition, biofilms are microbial populations that are attached to a surface, or to the surfaces of other organisms, and encase themselves in hydrated extracellular polymeric substance (EPS), which is also referred to as "slime". The chemical and physical properties of EPS will vary, but it is mainly composed of polysaccharides. EPS is also associated with other macromolecules such as proteins, DNA, lipids, and even humic substances (Nielsen et al., 1996, Tsuneda et al., 2003).

Even in the absence of pathogenic germs, it is extremely important for promoting and accelerating wound healing to remove coatings on the wound and thoroughly clean and disinfect the wound. Adhesions to the wound and resulting pain can be avoided by timely and thorough removal of wound coatings.

Various wound-treatment agents are known which on the one hand clean a wound and remove wound coatings and on the other hand kill off germs, without acting in an allergizing, sensitizing or tissue-damaging manner.

WO 03/004013 discloses a wound treatment agent that contains, in aqueous solution, polyhexamethylene biguanide ("PHMB") and at least one surfactant which is a glycine derivative, in particular a betaine, and/or a sulfosuccinate and/or an amide based on an unbranched fatty acid.

WO 94/27440 describes anti-infective PHMB agents with a mean molecular weight Mw of 2,900 to 15,000, in particular 3,200 to 5,000, which are used as an injury antiseptic and/or injury-treatment agent or as an antimicrobial, antiviral and/or antiparasitic agent, preferably by intravenous administration. Compared with previously known PHMB disinfectants, the PHMB disclosed here has a higher mean molecular weight Mw and exhibits an enhanced microbicidal action with lower toxicity. The anti-infective agent disclosed in WO 94/27440 exhibits reduced tissue damage as compared to other anti-infective agents, but its cleaning effect is low.

WO 2011/117384 describes a wound dressing comprising a polymer substrate and a composition comprising a) at least one antimicrobial active agent and b) an agent that reduces the cytotoxicity, comprising an oil-in-water emulsion that additionally has one or more alkanediols and/or one or more glyceryl ethers.

EP-A-1103264 describes a tissue cell growth-promoting solution comprising water-containing active oxygen as a prime ingredient which promotes the reconstruction of tissues, a process that corresponds to the last of the four main steps involved in wound healing biochemical processes: "blood vessel reaction", "blood vessel coagulation", "inflammation" and "reconstruction of tissues", which would otherwise have to rely on the natural healing power of the biobody itself.

EP-A-0601891 describes an antifungal and skin healing promoting agent comprising a hypochlorite, a sulfite, a nitrite, a chlorate, hydrogen peroxide, ozone water, a nitrate, and water.

WO 2007/099398 discloses compositions comprising (a) metasilicate, (b) carbonate, (c) gluconate, and (d) sulfate for the treatment of human skin to alleviate the symptoms of cosmetic or dermatologic skin conditions, including acne, rosacea and wrinkling caused by photodamage or conditions related to aging, hormonal imbalances, hyper-pigmentation, melasma, keratosis, dandruff, or the like. The compositions are also useful for removing biofilms from contact lenses and for inhibiting the growth of microbials that are correlated to the formation of biofilm in the oral cavity. Embodiments of the compositions of the latter uses are, e.g., dentifrices and mouth washes. The potential use of such compositions in wound treatment on the human body is neither taught nor suggested.

The technical problem underlying the invention is to provide a wound-treatment composition with a favorable cleaning effect which is suitable for cleaning wounds, dissolving incrustations and removing dressings and similar applications, which in combination with a common medicinal treatment, such as antibiotics treatment, has a curative effect on wound-healing and which is substantially free of undesirable side effects.

This object is achieved by the composition according to claim 1. Preferred embodiments are given in the sub-claims.

The invention therefore relates to a pharmaceutical composition for use as a wound-treatment agent which comprises (a) at least one silicate, (b) at least one carbonate, (c) at least one gluconate, and (d) at least one sulfate.

The compositions according to the invention may further comprise (e) at least one salt selected from alkali metal and/or alkaline earth metal sulfates, borates, bromides, citrates, acetates and lactates. The salt should not interfere with the biological activity of the composition. When other materials are present, the salt should not degrade those materials or interfere with their properties or biological activity. In other words, the salt should be inert with respect to the other components.

The compositions according to the invention show a remarkable and unexpected effect especially when used in aqueous solution as a rinsing agent for cleaning wounds. It has been surprisingly found that pretreatment of wounds with a composition according to the invention has a beneficial and synergistic effect on the traditional treatment of wounds with antibiotics or oxidizing agents in that it makes the latter treatment more effective. The composition is working on the EPS matrix by opening and loosening the debris, so that antibiotics are able to better penetrate and get in a position to effectively combat the infection. Chronic biofilm infections, such as endocarditis and osteomelitis, often persist indefinitely unless the infected material is removed. The invention however supports and improves the effect of antibiotics.

The compositions according to the invention are therefore especially suitable as rinsing or soaking agents for cleaning surfaces of a wound, for opening, loosening and removing biofilms from wound and other body surfaces, for dissolving incrustations or scabs from wound and other body surfaces and as dissolving agents for dressings.

Preferably, said at least one silicate is an alkali metal silicate selected from the group consisting of sodium or potassium metasilicate and sodium or potassium orthosilicate, and mixtures thereof, of which sodium metasilicate, in particular in the form of its pentahydrate is most preferred.

Preferably, said at least one carbonate is selected from the group consisting of sodium carbonate, sodium sesquicarbonate, sodium bicarbonate and mixtures thereof, of which sodium carbonate is most preferred.

Preferably, said at least one gluconate is selected from the group consisting of ammonium gluconate, lithium gluconate, sodium gluconate, sodium starch gluconate, potassium gluconate, ammonium acid gluconate, sodium acid gluconate, lithium acid gluconate, potassium acid gluconate, ammonium D-gluconate, lithium D-gluconate, sodium D-gluconate, potassium D-gluconate, gluconic acid, gluconic D-acid, gluconic L-acid, ammonium L-gluconate, lithium L-gluconate, sodium L-gluconate, potassium L-gluconate, calcium gluconate, calcium acid gluconate, calcium D-gluconate, calcium L-gluconate and mixtures thereof, of which sodium gluconate and potassium gluconate are most preferred.

Preferably, said at least one sulfate is selected from the group consisting of potassium aluminium sulfate, sodium sulfate, potassium sulfate, lithium sulfate, ammonium sulfate, magnesium sulfate, strontium sulfate, aluminium sulfate and mixtures thereof, of which potassium aluminium sulfate is most preferred.

The at least one salt defined in (e) may be a single salt material or a mixture of two or more salts alone. When the carrier matrix contains a mixture of salts, those salts are preferably present in equal amounts, e.g., a mixture of two salts in a 1:1 ratio.

Specific examples of suitable salts include, but are not limited to, sodium acetate, sodium bicarbonate, sodium borate, sodium bromide, sodium carbonate, sodium chloride, sodium citrate, sodium fluoride, sodium gluconate, sodium sulfate, calcium chloride, calcium lactate, calcium sulfate, potassium sulfate, tripotassium phosphate, potassium chloride, potassium bromide, potassium fluoride, magnesium chloride, magnesium sulfate and lithium chloride. Preferred salts are the inorganic salts, such as the Group 1 or 2 metal sulfates and chlorides. A particularly preferred salt, because of their low cost, is sodium chloride. Sodium chloride maybe substantially pure or in the form of rock salt, sea salt, or dendrite salt. Of these, Reef Crystals synthetic sea salt (Aquarium Systems, Inc.) is most preferred.

Silicates in the meta-forms of pentahydrates (e.g. Na₂SiO₃.5H₂O) are known in the art of cleaning technology as good builders with high pH, buffering capacity and dirt removal. Meta-silicates are known to prevent Ca precipitation. Silicates are unique among the oxy-anions, the common ionic 3D raster of SiO₄ is the tetrahedral.

Carbonates, exemplified by soda (Na₂CO₃), are known as effective builders with a high pH and effective buffering capacity. In very hard water precipitation may occur due to Ca and/or Mg carbonates. Soda performs well in dirt removal. Therefore, it is an active component in removal of extracellular Polymeric Substances (xPS) and soft not fully closed scaling.

Gluconates, exemplified by sodium or potassium gluconate, are known sequestrants. Combined with additives, such as sodium carbonates and/or meta-silicates, gluconates are active in scale removal from surfaces, including the skin.

Sulfates, exemplified by sodium sulfate, are known for their coagulation action.

In still a further aspect of the invention the compositions additionally comprise an emulsifying agent, a surfactant, a thickening agent, or a mixture thereof.

In a preferred embodiment of the invention the compositions further comprise a physiologically acceptable carrier, preferably selected from liposomes, solutions, in particular aqueous solutions, preferably with demineralized water, creams, emollients, ointments, gels, solid formulations and liquid formulations, or diluent.

In a most preferred embodiment of the invention the compositions are preferably used in aqueous solution at a pH which is preferably in the range of about 7.5 to about 9.

As stated before, the compositions according to the invention are especially suitable as rinsing or soaking agents for cleaning surfaces of a wound, for removing biofilms from wound and other body surfaces, for dissolving incrustations or scabs from wound and other body surfaces and as dissolving agents for dressings.

Without wanting to be bound to any scientific theory, the inventor believes that the surprising effects of the claimed compositions, in particular in cleaning surfaces of a wound and removing biofilms including glycocalyx produced by some bacteria, epithelia and other cells, may be explained as follows.

As biofilm formation starts from the first minute in aqueous environment, the first step is the formation of a conditioning film of organic molecules. After initial attachment, planktonic bacteria adhere irreversibly to the surface and proliferate to transform into bacterial micro-colonies. Subsequently bacteria generate a coating of xPS, which is essential for the development of the architecture of any biofilm matrix; it provides a framework into which microbial cells are inserted. Confocal laser scanning microscopy indicates that micro-colonies within a biofilm are three-dimensional structures of mushroom-like bacterial growth with water channels running between them for a constant supply of nutrients. The most part of the biofilm, generally around 97%, consists of water. The microbial cells form only about 2-5 % of the biofilm.

Briefly, according to the present invention, two compounds are selected for their builder activity, a meta-silicate and a carbonate. Both compounds creep behind the biofilm for loosening the xPS from the skin, and actually from the organic molecular layer on the skin. Together with two specific salts known for their coagulation and combined scale removing capabilities, a gluconate and a sulfate, the biofilm and existing scaling are removed with cleansing water.

Both the meta-silicate and carbonate are anionic in their action consisting of absorption to the xPS lowering the binding forces to their substrate. Bacteria in the xPS have positively charged cell walls as shown by Mera and Beveridge, J. Bacteriol 175 7:1936-1945 (1993). The original double layer of positive and negative molecules is loosened from the substrate by the meta-silicate and carbonate as they are negatively charged and creep under the xPS. Both meta-silicate and carbonate show to have synergistic action resulting in better cleaning as should be expected from the individual salts. This is expressed in the zeta potential which is still at the same level at some distance from the substrate surface compared to the situation with the xPS stuck directly in the wound. Zeta potential is defined as the electrical potential at the substrate surface with respect to the bulk liquid = water, i.e. the zeta is higher at the surface and becomes lower when the distance between substrate and biofilm or dirt increases. After addition of a composition according to the invention on the wound, it will seek the lowest potential energetic level. This basic rule in chemistry and physics is reflected in the situation of loosened and coagulated/encapsulated biofilm provided that the dosed concentrations are at the correct levels. In the end, the negatively charged organic layer or substrate is replaced by a negatively charged layer of the anionic salts composition of the invention.

In a further aspect of the present invention the composition comprises, in weight percent:
a) from about 0.1% to about 2.5%, preferably about 0.5% to about 2.0%, of a meta-silicate, preferably sodium meta-silicate;
b) from about 0.1% to about 2.0%, preferably from about 0.5% to about 1.5%, of a carbonate, preferably sodium carbonate;
c) from about 0.1% to about 1.0%, preferably from about 0.3% to about 0.7%, of a gluconate, preferably sodium gluconate;
d) from about 0.1% to about 1.0%, preferably from about 0.3% to about 0.7% of a sulfate, preferably potassium aluminium sulfate;
e) optionally, from about 0.1% to about 1.0%, preferably from about 0.3% to about 0.7% of a salt as defined herein, preferably sodium chloride, more preferably synthetic sea salt.

Preferably, the total percentage of ingredients in the composition is < 5 wt.%.

A particularly preferred composition according to the invention comprises:
- sodium metasilicate pentahydrate (PQ Silicas B.V., Netherlands), 1.83 wt.%;
- sodium carbonate (Tata Chemicals Europe, UK), 1.43 wt.%;
- sodium gluconate 99% (Jungbunzlauer S.A., France), 0.5 wt.%;
- potassium aluminium sulfate >99% (Kaliumaluin solid, Caldic Belgium N.V.), 0.5 wt.%;
- synthetic seasalt, containing 58% NaCl (Reef Crystals synthetic seasalt, Aquarium Systems, Inc.), 0.5 wt.%;
- in an aqueous solution, adding a suitable acid, for example citric acid or acetic acid or hydrochloric acid to adjust the pH to 7.5 to 9, in particular to about 8.

The concentration of the aqueous composition according to the invention for use as a wound-treatment agent may vary between ranges which are known in the art or can be derived from commercial wound-treatment agents, but is preferably in a dilution of about 1:200, i.e. 50 ml of stock solution in 10 liters of water.

The composition for use as a wound-treatment agent can principally be used in any form of preparation known and suitable for wound treatment, which are ointments, tinctures, sprays, rinsing solutions or a washing or shower gel for wound treatment, as a moisturizing gel or as a moist wound covering, as a dissolving gel for dissolving incrustations or scabs from body surfaces or wounds or for removing dressings and for changing moist dressings, and for treating burns and skin transplants.

Preferably, the compositions according to the invention are used as wound-treatment agent in the form of washing solutions, washing gels or gel-like wound coverings. For example, a wound-rinsing solution consists of 98 percent by weight of water, preferably 99.5 percent by weight of water.

When used in the form of a wound gel, a preferred composition contains 1 to 15 percent by weight of glycerine and 0.2 to 5 percent by weight of hydroxyethyl cellulose, especially 5 to 12 percent by weight of glycerine and 0.2 to 3 percent by weight of hydroxyethyl cellulose.

When the composition for use as a wound-treatment agent is used in the form of a wound gel, it contains the additives usually used in medical or cosmetic gels. For the compositions in accordance with the invention for use as a wound-treatment agent, additives of 1 to 15 percent by weight of glycerine and 0.2 to 5 percent by weight of hydroxyethyl cellulose, especially 5 to 12 percent by weight of glycerine and 0.2 to 3 percent by weight of hydroxyethyl cellulose, have proven to be advantageous for the mixtures as described above.

In all compositions in accordance with the present invention for use as wound-treatment agents, the additives and admixtures that are usually used for skin-cleaning, wound-cleaning and anti-infective agents can be present in addition to the mentioned components. Other preservatives, colorants, flavors and fragrances, binding agents, moisturizing or wetting agents, consistency regulators, solubilizers or the like can be mentioned, for example which can be added in the usual quantities.

It is preferable however to keep the number and concentration of the additives and admixtures as low as possible or to avoid the same entirely. It is understood that substances which lead to a reduced effect of the components already present or have a disadvantageous effect on the tissue to be cleaned shall be avoided as far as possible.

Either cleaned water or another aqueous solution as used otherwise in the field of medicine is suitable as a solvent in the compositions according to the invention for use as a wound-treatment agent. Suitable solvents are for example a saline or Ringer's solution. A saline solution of 0.4 to 1.2 percent of weight can be used for example. The saline or Ringer's solution is used especially preferably in a physiological dilution. In the case of solutions, the invention comprises both the solutions readily diluted and concentrates which need to be diluted prior to use.

The composition in accordance with the invention for use as a wound-treatment agent is especially suitable for treating chronic wounds, as occur for example in diabetics or bedridden patients. Diluted aqueous solutions can be used for rinsing or bathing wounds or for soaking and humidifying wound coverings. Adhering wound dressings could be removed easily from the wound by softening with the solution without damaging the wound tissue. When dressings are removed it often occurs that incrustations already formed are torn open again in an undesirable manner during the removal of the dressing, thus leading to delayed healing of the wound. The composition in accordance with the invention for use as a wound-treatment agent provides a remedy for this purpose too. When the composition in accordance with the invention for use as a wound-treatment agent is applied to the dressing, it penetrates and detaches the same from the incrustations on the wound, so that the dressing can be removed without damaging any incrustations already formed. At the same time, it is prevented that bacteria or the like are implanted in the wound.

The composition in accordance with the invention for use as a wound-treatment agent in gel form can be used as a moisturizing gel or moisture wound covering. Wound coatings can thus be detached, the wound can be cleaned. One application is the removal of scabs in patients' noses who are artificially respirated. The gel is also especially suitable for treating bums and skin transplants in order to keep the wound humid and smooth and to prevent forming breeding ground for bacteria and other germs. Application can be made directly on the wound or on a wound dressing. The gel layer reliably prevents recontamination of disinfected wounds by germs introduced from the outside.

The main field of application of the composition according to the invention for use as a wound-treatment agent is, as mentioned before, the cleaning of wounds, wound conditioning and wound remediation. Main focus must be laid on the removal of wound coatings which delay the healing of wounds and form a breeding ground for the new settlement of pathogenic germs on non-infected wounds.

Traditionally focus on planktonic cells in wound healing is outdated science. The focus of the composition according to the invention is based on wound bioburden. Naturally occurring bacteria attached to surfaces rarely behave like planktonic bacteria. Up to 80% of human infectious diseases are biofilm related (NIH). The remaining 20% are microbial cells that reside within a microbial community encased within the EPS matrix. Antibiotics fail to eradicate bioflms due to poor penetration, metabolic inhibition and the immune response to antimicrobials.

The composition according to the invention provides for the first time a composition for use as a wound-treatment agent which combines a favorable cleaning effect as a pretreatment of wounds with the traditional treatment of wounds with antibiotics or oxidizing agents, e.g. a peroxide ointment, in that it makes the latter treatment more effective, without leading to irritations or even destruction of the tissue. Wounds are reliably cleaned, odor formation is suppressed, and wound healing is promoted.

The invention will now be explained in further detail with reference to the following examples.

### Example 1

In this example it is demonstrated that a preferred composition according to the invention, hereinafter named "Aqua Finesse" is effective in the treatment of biofilms in vitro and in chronic wounds.

Aqua Finesse had the same composition as the preferred composition disclosed in the general description:
- sodium metasilicate pentahydrate, 1.83 wt.%;
- sodium carbonate, 1.43 wt.%;
- sodium gluconate (99%), 0.5 wt.%;
- potassium aluminium sulfate (>99%), 0.5 wt.%;
- synthetic seasalt (containing 58% NaCl), 0.5 wt.%;
in an aqueous solution, to which citric acid was added to adjust the pH to 8.0.

The following strains of bacteria were tested on treatment with "Aqua Finesse":
- *Staphylococcus aureus,*
- *Staphylococcus epidermidis*
- *Enterococcus faecalis*
- *Proteus vulgaris*
- *Enterobacter cloacae*

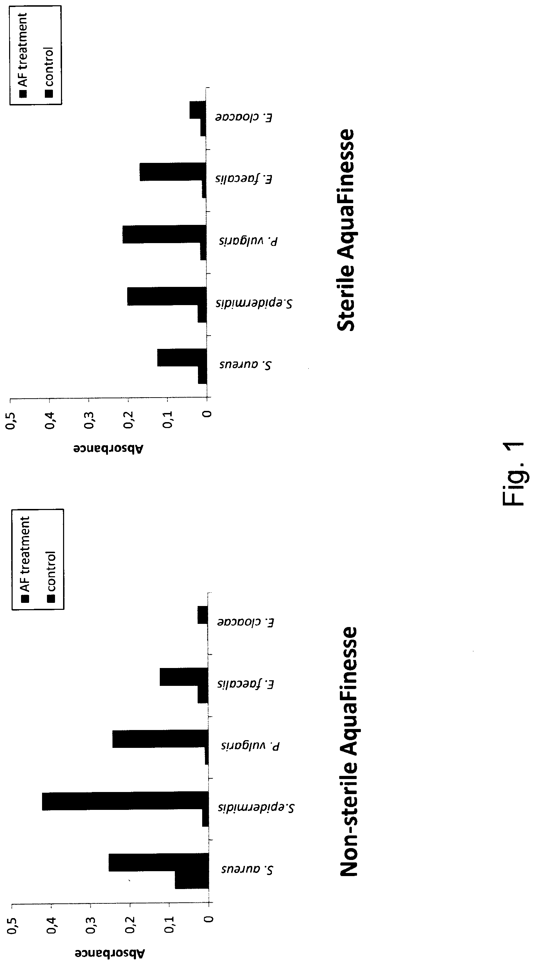

These bacteria are all to be found in open wounds. Many infections are polymicrobial. The results showed that "Aqua Finesse", both as a sterile and non-sterilized product, performs efficiently on the biofilms. See Figure 1

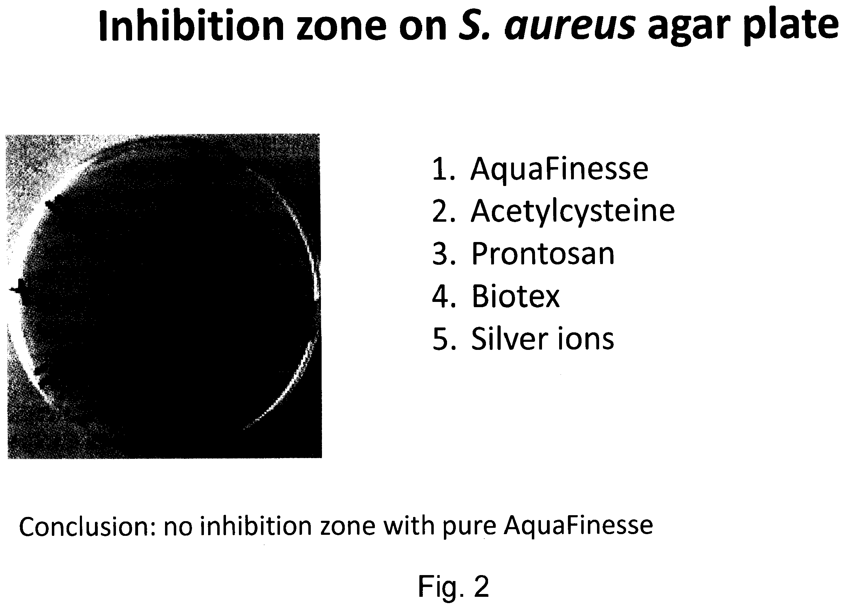

Pure "Aqua Finesse" was applied on S. aureus agar plate to determine its possible biocidal effect. However, no inhibition zone was found. See Figure 2.

A slime-test was performed with the above five bacteria strains and mixtures thereof, and with *Pseudomonas aeruginosa* at two different doses of "Aqua Finesse" to assess if it dissolved the "slime" layer of the biofilm. All these tests proved that "Aqua Finesse" had effect in removing the slime layer and making biofilms susceptible to antibiotics.

An MTT assay was performed with the separate bacteria biofilms and a biofilm of the mixture exposed to 3 different doses of "Aqua Finesse" for 5 and 30 minutes. An MTT assay was also performed to assess the effectiveness of treatment with a combination of "Aqua Finesse" and antibiotics. Afterwards a confocal laser scanner microscope was used. It was demonstrated that antibiotic treatment is more effective after pre-treatment with "AquaFinesse".

It was further surprising to note that the test with S. *aureus* showed a biofilm left, but it became susceptible for antibiotics. This bacteria is normally resistant against antibiotics. It was concluded that removing the outer layer of the biofilm may be sufficient for a successful treatment of the bacteria with antibiotics.

An MTT assay showed that the metabolic activity of the bacteria does not change when exposed to "Aqua Finesse" in different concentrations. Performing multiple slime-tests showed that treatment with "Aqua Finesse" partially opens and loosens biofilm slime and resulted in fewer biofilm clumps.

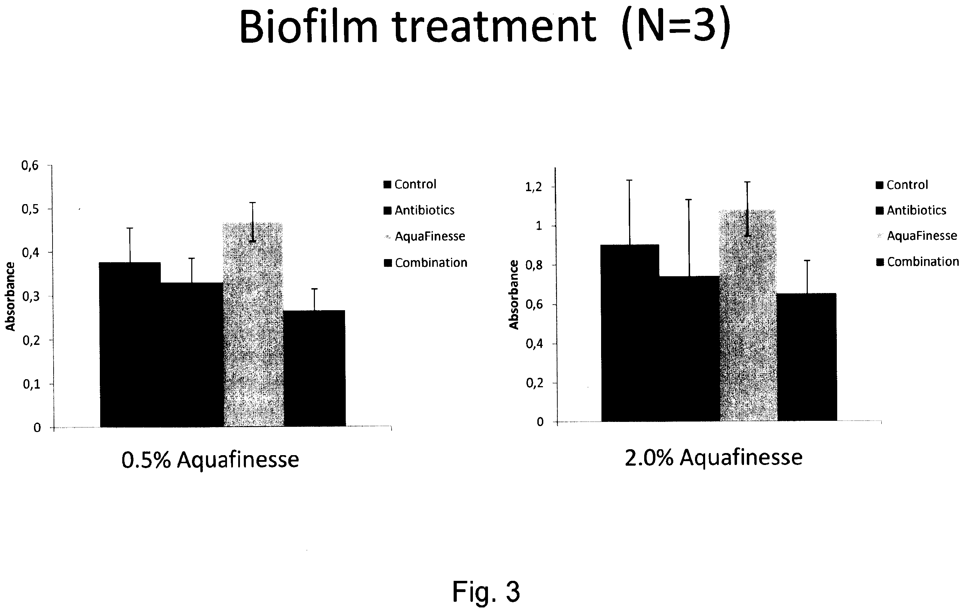

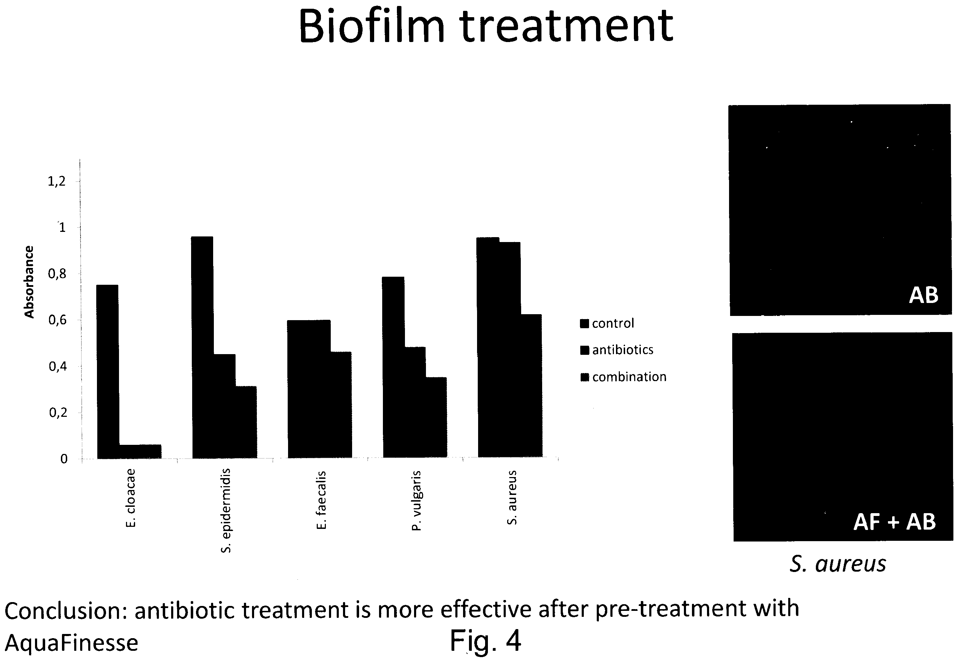

MTT assay analysis showed prophylactic use of "Aqua Finesse" to have no noticeable effect. Furthermore treatment of biofilms with only "Aqua Finesse" or antibiotics showed no significant improvement, whereas treatment with "Aqua Finesse" followed by antibiotic treatment showed significantly better results. See Figs. 3 and 4.

Confocal laser scanning microscopy showed that the biofilm thickness of the mixture of *S*. *aureus and Ps. Aeruginosa* treated only with "Aqua Finesse" decreased the least as compared to the control group. The biofilm exposed to antibiotics and the combination of "Aqua Finesse" and antibiotics showed a reduction in thickness. The CLSM confirmed that the combination of "Aqua Finesse" and antibiotics was the most effective against the biofilms by showing more dead bacteria, although it did not completely remove the biofilm. Although some biofilm was still present it was not attached to the surface anymore.

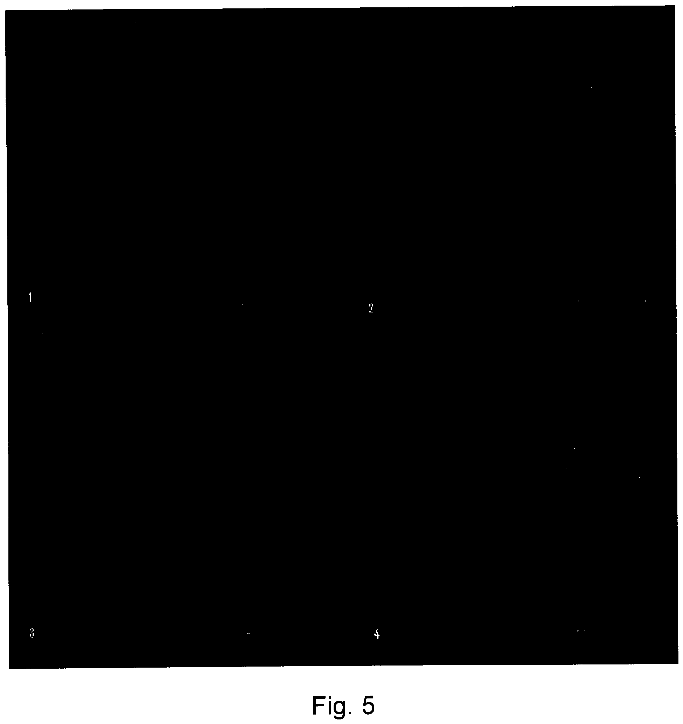

The mixed biofilm of *S*. *aureus* and *Pseudomonas* was used for the Confocal laser scanning microscopy (CLSM). The biofilm was tested in 4 different conditions. The first well was filled with an untreated biofilm of the mixture as control. In the second well antibiotics were added to the biofilm. "Aqua Finesse" was added to the third well. The fourth well was treated with a combination of "Aqua Finesse" and the antibiotics. Figure 5 shows the CLSM of the biofilm in the different conditions. The green dots show the living cells and the red dots show the death cells. The picture shows that "Aqua Finesse" in combination with the antibiotic kills many cells.

### Example 2

A stock solution of a composition according to the present invention was made as follows.

80 liters of demineralized water (Pharmaline) were loaded into a vessel, to which the following ingredients were added under continuous stirring to prepare a 50% stock solution:
- Artificial sea salt (reef Crystals) 200 g
- Sodium gluconate 200 g
- Potassium aluminium sulfate ("kalialuin) 200 g
- Sodium carbonate 573 g
- Sodium metasilicate 733 g

This solution was made sterile by gamma irradiation and used to fill 2 litre PE bottles, which were then sealed and provided with a batch number.

### Example 3

Cytotoxicity of the solution prepared in Example 2, hereinafter also named Solution A, is assessed by three independent methods in four different human fibroblast cell lines. Cell viability is determined using the Alamar Blue cell viability stain, the percentage of surviving cells after exposure is determined by cell count and the ability of cells to survive and recover after exposure is assessed using a clonal survival assay. Cells were exposed to the test solution for 30 minutes and cytotoxicity was determined 24 hr and 48 hr after exposure. All assays are performed in three independent tests.

Exposure of human fibroblasts to the tested Solution A resulted in a decreased cell survival in some of the cytotpxicity test, indicating cytotoxic properties of the solution. Cytotoxicity was observed in both primary and immortalized human fibroblast cell lines and was most pronounced in the clonal cell survival. Cytotoxicity was also observed for the immortalized MRC-5 lung fibroblasts (Huschtscha and Holliday, 1983) and VH-25 primary skin fibroblasts (Abrahams et al., 1992) in the Alamar Blue viability stain for the MRC-5 cells in the cell count assay.

It was concluded that Solution A can induce cytotoxicity in cultured human fibroblasts, but the cytotoxic potency strongly depends on the cell type.

### Example 4

The efficacy of Composition A to biofilms was experimentally tested in a static system with different concentrations of the composition in sterile and non-sterile form (50%, 25%, 10%, 5%, 1%, 0.5% and 0.1% solutions in sterile MQ) .

The following biofilm-generating microorganism strains were used: *S. aureus* ATCC 6538 and MRSA S-325, and *S*. *epidermis* 1457 and 0068.

It was found that both *S*. *epidermis* strains formed more extensive biofilms than the *S. aureus* strains. However, for none of the concentrations of the tested compositions a significant reduction in the reduction of the amount of biofilm was found, neither for the treatment of 10 minutes, nor for the treatment of 30 minutes.

Of the four tested strains, *S. aureus* MRSA S-325 formed the least extensive biofims, but also with this strain no significant reduction of the amount of biofilm was observed at any of the concentrations of the tested composition, neither for the treatment of 10 minutes, nor for the treatment of 30 minutes. However, for *S. aureus* ATCC 6538 a clear and significant reduction was observed for the treatment of 10 minutes and 30 minutes at the 25%, 10%, 25%, 10% and 5% concentrations in a decreasing order with decreasing concentrations. No difference in efficiency was found between the sterile and non-sterile solutions. A longer treatment did not result in this case in an increase in the number of effective concentrations.

The dosage which showed clear effects on the specific biofilm of a bacterial strain (*S. aureus* ATCC 6538) did not result in cytotoxic effects on the human fibroblast cell cultures.

## Claims

1. A pharmaceutical composition for use in treating or cleaning a wound on the human body and removing a biofilm from a surface of the wound, comprising (in weight percent):
(a) from about 0.1% to about 2.5% of a meta-silicate,
(b) from about 0.1% to about 2.0% of a carbonate,
(c) from about 0.1% to about 1.0% of a gluconate,
(d) from about 0.1% to about 1.0% of potassium aluminium sulfate, and
(e) from about 0.1% to about 1.0% of sodium chloride;
wherein the use comprises applying said composition to a wound, optionally followed by applying a curative agent to the wound

2. A composition for use according to claim 1, wherein the sodium chloride is synthetic sea salt.

3. A composition for use according to claim 1 or claim 2, wherein the composition is in the form of an aqueous solution having a pH of 7.5 - 9.

4. A composition for use according to any one of claims 1 to 3, wherein said meta-silicate is an alkali metal silicate selected from the group consisting of sodium or potassium meta-silicate, and mixtures thereof.

5. A composition for use according to any of of claims 1 to 4, wherein said carbonate is selected from the group consisting of sodium carbonate, sodium sesquicarbonate, sodium bicarbonate, and mixtures thereof.

6. A composition for use according to any one of claims 1 to 5, wherein said gluconate is selected from the group consisting of ammonium gluconate, lithium gluconate, sodium gluconate, sodium starch gluconate, potassium gluconate, ammonium acid gluconate, sodium acid gluconate, lithium acid gluconate, potassium acid gluconate, ammonium D-gluconate, lithium D-gluconate, sodium D-gluconate, potassium D-gluconate, gluconic acid, gluconic D-acid, gluconic L-acid, ammonium L-gluconate, lithium L-gluconate, sodium L-gluconate, potassium L-gluconate, calcium gluconate, calcium acid gluconate, calcium D-gluconate, calcium L-gluconate, and mixtures thereof.

7. A composition for use according to claim 1, wherein the composition comprises (in weight percent):
a) from about 0.5% to about 2.0% of sodium meta-silicate;
b) from about 0.5% to about 1.5% of sodium carbonate;
c) from about 0.3% to about 0.7% of sodium gluconate;
d) from about 0.3% to about 0.7% of potassium aluminium sulfate;
e) from about 0.3% to about 0.7% of sodium chloride.

8. A composition for use according to claim 7, wherein the sodium chloride is synthetic sea salt.

9. A composition for use according to any one of claims 1 to 8, wherein the composition further comprises an emulsifying agent, a surfactant, a thickening agent, or a mixture thereof.

10. A composition for use according to any one of claims 1 tot 9, wherein the composition further comprises a physiologically acceptable carrier.

11. A composition for use according to claim 10, wherein the physiologically carrier is selected from liposomes, solutions, preferably aqueous solutions, creams, emollients, ointments, gels, solid formulations and liquid formulations, or diluents.

12. A compositon for use according to any one of claims 1 to 11, wherein the composition is in the form of a liquid, cream, oil, gel, fluid cream, lotion, emulsion or microemulsion.

13. A composition for use according to any one of claims 1 to 12, wherein the composition is in the form of a wound-rinsing solution, which consists of at least 98 percent by weight of water or demineralized water.

14. A composition for use according to any one of claims 1 to 13, wherein the composition is in the form of a wound gel, which contains 1 to 15 percent by weight of glycerine and 0.2 to 5 percent by weight of hydroxyethyl cellulose, especially 5 to 12 percent by weight of glycerine and 0.2 to 3 percent by weight of hydroxyethyl cellulose; or in the form of a rinsing solution or a washing or shower gel for wound treatment, or a moisturizing gel or a moist wound covering, or a dissolving gel for dissolving incrustations or scabs from body surfaces or wounds or for removing dressings or for changing moist dressings, or for treating burns and skin transplants.

15. A composition for use according to any one of claims 1 to 14, wherein the curative agent comprises at least one of an antimicrobial agent and an oxidizing agent, the oxidizing agent preferably being a peroxide ointment, wherein the composition preferably enhances effectiveness of the antibiotic or the oxidizing agent when it is applied to the wound.

## Patentansprüche

1. Pharmazeutische Zusammensetzung zur Verwendung bei der Behandlung oder Reinigung einer Wunde am menschlichen Körper und zur Entfernung eines Biofilms von einer Oberfläche der Wunde, umfassend (in Gewichtsprozent):
(a) von etwa 0,1 % bis etwa 2,5 % eines Meta-Silikats,
(b) von etwa 0,1 % bis etwa 2,0 % eines Carbonats,
(c) von etwa 0,1 % bis etwa 1,0 % eines Gluconats,
(d) von etwa 0,1 % bis etwa 1,0 % Kaliumaluminiumsulfat, und
(e) von etwa 0,1 % bis etwa 1,0 % Natriumchlorid;
wobei die Verwendung das Auftragen der Zusammensetzung auf eine Wunde umfasst, gegebenenfalls gefolgt vom Auftragen eines heilenden Mittels auf die Wunde

2. Zusammensetzung zur Verwendung gemäß Anspruch 1, wobei das Natriumchlorid synthetisches Meersalz ist.

3. Zusammensetzung zur Verwendung nach Anspruch 1 oder Anspruch 2, wobei die Zusammensetzung in Form einer wässrigen Lösung mit einem pH-Wert von 7,5 bis 9 vorliegt.

4. Zusammensetzung zur Verwendung nach einem der Ansprüche 1 bis 3, wobei das Metasilikat ein Alkalimetallsilikat ist, ausgewählt aus der Gruppe bestehend aus Natrium- oder Kaliummetasilikat und Mischungen davon.

5. Zusammensetzung zur Verwendung nach einem der Ansprüche 1 bis 4, wobei das Carbonat aus der Gruppe ausgewählt ist, die aus Natriumcarbonat, Natriumsesquicarbonat, Natriumbicarbonat und Mischungen davon besteht.

6. Zusammensetzung zur Verwendung nach einem der Ansprüche 1 bis 5, wobei das Gluconat aus der Gruppe bestehend aus Ammoniumgluconat, Lithiumgluconat, Natriumgluconat, Natriumstärkegluconat, Kaliumgluconat, Ammoniumsäuregluconat, Natriumsäuregluconat, Lithiumsäuregluconat, Kaliumsäuregluconat, Ammonium-D-gluconat, Lithium-D-gluconat, Natrium-D-gluconat, Kalium-D-gluconat, Gluconsäure, Glucon-D-Säure, Glucon-L-Säure, Ammonium-L-Gluconat, Lithium-L-Gluconat, Natrium-L-gluconat, Kalium-L-gluconat, Calciumgluconat, Calciumsäuregluconat, Calcium-D-gluconat, Calcium-L-gluconat und Mischungen davon.

7. Zusammensetzung zur Verwendung nach Anspruch 1, wobei die Zusammensetzung (in Gewichtsprozent) umfasst:
a) etwa 0,5 % bis etwa 2,0 % Natriummetasilikat;
b) etwa 0,5 % bis etwa 1,5 % Natriumcarbonat;
c) etwa 0,3 % bis etwa 0,7 % Natriumgluconat;
d) etwa 0,3 % bis etwa 0,7 % Kaliumaluminiumsulfat;
e) etwa 0,3 % bis etwa 0,7 % Natriumchlorid.

8. Zusammensetzung zur Verwendung nach Anspruch 7, wobei das Natriumchlorid synthetisches Meersalz ist.

9. Zusammensetzung zur Verwendung nach einem der Ansprüche 1 bis 8, wobei die Zusammensetzung außerdem einen Emulgator, ein Tensid, ein Verdickungsmittel oder eine Mischung davon umfasst.

10. Zusammensetzung zur Verwendung nach einem der Ansprüche 1 bis 9, wobei die Zusammensetzung außerdem einen physiologisch verträglichen Träger umfasst.

11. Zusammensetzung zur Verwendung nach Anspruch 10, wobei der physiologische Träger ausgewählt ist aus Liposomen, Lösungen, vorzugsweise wässrigen Lösungen, Cremes, Weichmachern, Salben, Gelen, festen Formulierungen und flüssigen Formulierungen oder Verdünnungsmitteln.

12. Zusammensetzung zur Verwendung nach einem der Ansprüche 1 bis 11, wobei die Zusammensetzung in Form einer Flüssigkeit, Creme, eines Öls, eines Gels, einer flüssigen Creme, einer Lotion, einer Emulsion oder einer Mikroemulsion vorliegt.

13. Zusammensetzung zur Verwendung nach einem der Ansprüche 1 bis 12, wobei die Zusammensetzung in Form einer Wundspüllösung vorliegt, die zu mindestens 98 Gewichtsprozent aus Wasser oder demineralisiertem Wasser besteht.

14. Zusammensetzung zur Verwendung nach einem der Ansprüche 1 bis 13, wobei die Zusammensetzung in Form eines Wundgels vorliegt, das 1 bis 15 Gewichtsprozent Glycerin und 0,2 bis 5 Gewichtsprozent Hydroxyethylcellulose, insbesondere 5 bis 12 Gewichtsprozent Glycerin und 0.2 bis 3 Gewichtsprozent Hydroxyethylcellulose enthält; oder in Form einer Spüllösung oder eines Wasch- oder Duschgels zur Wundbehandlung oder eines Feuchtigkeitsgels oder einer feuchten Wundabdeckung oder eines Auflösegels zum Lösen von Verkrustungen oder Schorf von Körperoberflächen oder Wunden oder zum Entfernen von Verbänden oder zum Wechseln von Feuchtverbänden oder zur Behandlung von Verbrennungen und Hauttransplantationen.

15. Zusammensetzung zur Verwendung nach einem der Ansprüche 1 bis 14, wobei das Heilmittel mindestens eines von einem antimikrobiellen Mittel und einem Oxidationsmittel umfasst, wobei das Oxidationsmittel vorzugsweise ein Peroxidsalbe ist, wobei die Zusammensetzung vorzugsweise die Wirksamkeit des Antibiotikums oder des Oxidationsmittels erhöht, wenn sie auf die Wunde aufgetragen wird.

## Revendications

1. Composition pharmaceutique destinée à être utilisée pour traiter ou nettoyer une plaie sur le corps humain et éliminer un biofilm d'une surface de la plaie, comprenant (en pourcentage en poids) :
(a) d'environ 0,1 % à environ 2,5 % d'un méta-silicate,
(b) d'environ 0,1 % à environ 2,0 % d'un carbonate,
(c) d'environ 0,1 % à environ 1,0 % d'un gluconate,
(d) d'environ 0,1 % à environ 1,0 % de sulfate d'aluminium et de potassium, et
(e) d'environ 0,1 % à environ 1,0 % de chlorure de sodium ;
l'utilisation comprenant l'application de ladite composition sur une plaie, éventuellement suivie de l'application d'un agent curatif sur la plaie

2. Composition à utiliser selon la revendication 1, dans laquelle le chlorure de sodium est du sel marin synthétique.

3. Composition à utiliser selon la revendication 1 ou la revendication 2, dans laquelle la composition est sous la forme d'une solution aqueuse ayant un pH de 7,5 à 9.

4. Composition à utiliser selon l'une quelconque des revendications 1 à 3, dans laquelle ledit méta-silicate est un silicate de métal alcalin choisi dans le groupe constitué du méta-silicate de sodium ou de potassium, et de leurs mélanges.

5. Composition à utiliser selon l'une quelconque des revendications 1 à 4, dans laquelle ledit carbonate est choisi dans le groupe constitué du carbonate de sodium, du sesquicarbonate de sodium, du bicarbonate de sodium et de leurs mélanges.

6. Composition à utiliser selon l'une quelconque des revendications 1 à 5, dans laquelle ledit gluconate est choisi dans le groupe constitué du gluconate d'ammonium, du gluconate de lithium, du gluconate de sodium, du gluconate d'amidon sodique, du gluconate de potassium, du gluconate acide d'ammonium, du gluconate acide de sodium, gluconate acide de lithium, gluconate acide de potassium, D-gluconate d'ammonium, D-gluconate de lithium, D-gluconate de sodium, D-gluconate de potassium, acide gluconique, D-acide gluconique, L-acide gluconique, L-gluconate d'ammonium, L-lithium le gluconate, le L-gluconate de sodium, le L-gluconate de potassium, le gluconate de calcium, le gluconate acide de calcium, le D-gluconate de calcium, le L-gluconate de calcium et leurs mélanges.

7. Composition à utiliser selon la revendication 1, dans laquelle la composition comprend (en pourcentage en poids) :
a) d'environ 0,5 % à environ 2,0 % de métasilicate de sodium ;
b) d'environ 0,5 % à environ 1,5 % de carbonate de sodium ;
c) d'environ 0,3 % à environ 0,7 % de gluconate de sodium ;
d) d'environ 0,3 % à environ 0,7 % de sulfate d'aluminium et de potassium ;
e) d'environ 0,3 % à environ 0,7 % de chlorure de sodium.

8. Composition à utiliser selon la revendication 7, dans laquelle le chlorure de sodium est du sel marin synthétique.

9. Composition à utiliser selon l'une quelconque des revendications 1 à 8, dans laquelle la composition comprend en outre un agent émulsifiant, un tensioactif, un agent épaississant ou un mélange de ceux-ci.

10. Composition à utiliser selon l'une quelconque des revendications 1 à 9, dans laquelle la composition comprend en outre un véhicule physiologiquement acceptable.

11. Composition à utiliser selon la revendication 10, dans laquelle le support physiologique est choisi parmi les liposomes, les solutions, de préférence les solutions aqueuses, les crèmes, les émollients, les pommades, les gels, les formulations solides et les formulations liquides, ou les diluants.

12. Composition à utiliser selon l'une quelconque des revendications 1 à 11, dans laquelle la composition se présente sous la forme d'un liquide, d'une crème, d'une huile, d'un gel, d'une crème fluide, d'une lotion, d'une émulsion ou d'une micro-émulsion.

13. Composition à utiliser selon l'une quelconque des revendications 1 à 12, dans laquelle la composition est sous la forme d'une solution de rinçage des plaies, qui est constituée d'au moins 98 pour cent en poids d'eau ou d'eau déminéralisée.

14. Composition pour utilisation selon l'une quelconque des revendications 1 à 13, dans laquelle la composition se présente sous la forme d'un gel pour plaies, qui contient de 1 à 15 pour cent en poids de glycérine et de 0,2 à 5 pour cent en poids d'hydroxyéthyl-cellulose, en particulier de 5 à 12 pour cent en poids de glycérine et de 0.2 à 3 pour cent en poids d'hydroxyéthylcellulose; ou sous forme de solution de rinçage ou de gel de lavage ou de douche pour le traitement des plaies, ou de gel hydratant ou de couverture humide des plaies, ou de gel dissolvant pour dissoudre les incrustations ou les croûtes de la surface du corps ou des plaies ou pour enlever les pansements ou pour changer les pansements humides, ou pour traiter les brûlures et les greffes de peau.

15. Composition à utiliser selon l'une des revendications 1 à 14, dans laquelle l'agent curatif comprend au moins un agent antimicrobien et un agent oxydant, l'agent oxydant étant de préférence une pommade au peroxyde, la composition améliorant de préférence l'efficacité de l'antibiotique ou de l'agent oxydant lorsqu'elle est appliquée sur la plaie.
